# EUROPEAN PATENT APPLICATION

(11) **EP 1 376 134 A1**
(43) Date of publication of application: **02.01.2004**
(21) Application number: 02014441.6
(22) Date of filing: 28.06.2002
(51) Int. Cl.: G01N 33/68

(54) **A method of screening for TRAIL-inhibitors**

(71) Applicant: Universitätsklinikum Charité Medizinische Fakultät der Humboldt-Universität zu Berlin, 10117 Berlin (DE)
(72) Inventor: Aktas, Orhan, Dr., AG Klin. Neuroimmunologie, Schumannstrasse 20/21, 10117 Berlin (DE); Zipp, Frauke, Dr. Prof., AG Klin. Neuroimmunologie, Schumannstrasse 20/21, 10117 Berlin (DE)
(74) Representative: Goddar, Heinz J., Dr.

(57) **Abstract**

The application relates to a method of screening for compounds capable of inhibiting TRAIL to induce neuronal cell injury, to compounds identified by this method and to the use of such compounds.

## Description

This invention relates to a method of screening for compounds capable of inhibiting tumor necrosis factor-related apoptosis inducing ligand (TRAIL) to induce neuronal cell injury, compounds identified by this method and uses of such compounds.

### Background of the invention

Multiple sclerosis (MS) is a chronic inflammatory disease of the central nervous system (CNS) and is the major cause of neurological disability in young adults in Western countries (Noseworthy *et al.* 2000, *N. Engl. J. Med.* **343**, 938-952). The etiology of MS remains unclear but an autoimmunological genesis has been supected and is now widely accepted.

The course of MS is accompanied by an attack of the immune system against myelin basic proteins during which myelin-specific T cells invade the CNS and promote demyelination of the axons. As a result, axonal cell death occurs and axonal loss was found to be an early event as demonstrated in an experimental animal model for MS, referred to as "experimental autoimmune encephalomyelitis" (EAE) (Ferguson *et al.* 1997, *Brain* **120**, 393-399; Kornek *et al.* 2000, *Am. J. Pathol.* **157**, 267-276). Furthermore, it became evident that in patients with MS long term neurological disability correlates rather with irreversible injury to neuronal structures such as axonal loss than with the degree of demyelination (Trapp *et al.* 1998, *N. Engl. J Med.* **338**, 278-285; Coles *et al.* 1999, *Ann. Neurol.* **46**, 296-304; Noseworthy *et al.* 2000, *N. Engl. J. Med.* **343**, 938-952).

Apototic death of T cells plays a critical role in the maintainance of self tolerance (Lenardo *et al.* 1999, *Annu. Rev. Immunol.* **17**, 221-253; Pender 1999, *Immunol. Cell Biol.* **77***,* 216-223) and seems to be misregulated in autoimmune diseases. There is evidence for the pathogenic and therapeutic role of CD95 ;(APO1/Fas)-mediated T cell apoptosis in MS (Dowling *et al.* 1996, *J. Exp. Med.* **184,** 1513-1518; D'Souza *et al.* 1996, *J. Exp. Med.* **184**, 2361-2370; Zipp *et al.* 1999, *Immunol. Today* **20**, 550-554).

Besides the CD95 receptor/ligand system, several other apoptosis-associated receptor/ligand interactions were found to be involved in the fate of a cell, in particular the recently described sysstem of tumor necrosis factor (TNF)-related apoptosis-inducing ligand (TRAIL) and its receptor. TRAIL (also referred to as APO-2 ligand) (Wiley *et al.* 1995, *Immunity* **3***,* 673-682; Pitti *et al.* 1996, *J. Biol. Chem.* **271**, 12687-12690) is a member of the TNF/nerve growth factor superfamily acting via several receptors thought to induce or block apoptosis. Until now, the group of TRAIL receptors comprises the death-inducing receptors TRAIL receptor 1 (TRAIL-R1; also referred to as DR4) and TRAIL-R2 (DR5), and the truncated TRAIL receptors TRAIL-R3 (DcR1) and TRAIL-R4 (DcR2) (*for review see* Degli-Esposti 1999, *J. Leukoc. Biol.* **65***,* 535-542).

Cell death induced by TRAIL was initially believed to be restricted to malignant tumor cells. Thus, TRAIL was proposed to be an excellent therapeutic for cancer treatment without the common side effects (Walczak *et al.* 1999, *Nat. Med.* **5***,* 157-163). Concerns were raised, however, since an induction of apoptosis by TRAIL in cultured normal human hepatocytes was shown (Jo *et al.* 2000, *Nat. Med.* **6***,* 564-567). Thus, TRAIL was suspected to exhibit adverse side effects such as liver toxicity if used systemically (Jo *et al.* 2000, *Nat. Med.* **6***,* 564-567).

By investigating living brain slices, it could previously be shown that TRAIL is able to induce massive neuronal cell destruction in (normal tissue of) the human brain (Kitsch *et al.* 2000, *Lancet* **356**, 827-828). In contrast to the CD95 system, peripheral control of the human myelin-specific T cells is not mediated by TRAIL-induced apoptosis (Wendling *et al.* 2000, *Cell Death Differ* **7**, 564-567). Upregulation of TRAIL on stimulation of these cells, however, suggests a role for the TRAIL system in the destructive effects of T cells in brain inflammation. In fact, TRAIL itself is not expressed in adult human brain tissue, but both apoptosis-mediating and truncated TRAIL receptors are present (Dörr *et al.* 2000, *J*. *Neurosci.* **22:RC209,** 1-5).
Although several lines of evidence point to a potential role of disturbances in apoptosis-associated receptor/ligand interactions in the etiology of MS, no clear-cut picture has emerged. Accordingly, identification of a promising treatment of MS has been hampered. Therefore there exists a need in the art to identify means that facilitate treatment of MS and, when applied, allow for an amelioration of MS-related symptoms. It is therefore an object of the present invention to provide means that allow for an identification of compounds useful in the treatment of MS.

This object is solved by a method of screening for compounds capable of inhibiting tumor necrosis factor-related apoptosis inducing ligand (TRAIL) to induce neuronal cell injury comprising the steps:
a) contacting TRAIL with a compound/compounds/a combinatorial library of compounds suspected of inhibiting TRAIL,
b) detecting an interaction of TRAIL with a compound/compounds.
   Preferably the method according to the present invention further comprises the step
c) selecting those compounds that detectably interact with TRAIL.
In a preferred embodiment, detection occurs by a method selected from the group comprising fluorimetry, surface plasmon resonance, gel filtration chromatography, mass spectrometry and nuclear magnetic resonance (NMR).

The object of the present invention is also solved by a method of screening for compounds capable of interfering with the interaction of TRAIL and its receptor(s) comprising the steps:
a) Contacting cells which are receptive for TRAIL-mediated apoptosis with a compound suspected of interfering with the interaction of TRAIL with its receptors) in the presence of a TRAIL-mediated apoptosis inducing agent,
b) measuring an apoptotic response.
   In a preferred embodiment the method according to the present invention further comprises the steps:
c) Contacting cells of the same type as in a) but not having undergone the contacting step a), with a soluble TRAIL-receptor-fusion protein in the presence of a TRAIL-mediated apoptosis inducing agent,
d) measuring an apoptotic response,
e) comparing the apoptotic response of step b) with the apoptotic response of step d).
In one embodiment, the cells contacted in steps a) and c) are cell slices of tissue, cell suspensions and/or cells grown on a surface, wherein preferably the surface is an artificial surface, and, even more preferred the surface of a membrane of a bioreactor.

In one embodiment, the soluble TRAIL-receptor fusion protein is selected from the group comprising TRAIL-R1 :Fc, TRAIL-R2:Fc, TRAIL-R3:Fc, TRAIL-R4:Fc and OPG:Fc.

Preferably, the cells which are receptive for TRAIL-mediated apoptosis are selected from the group comprising neuronal cell lines, glioma cell lines, in particular LN18 cells and T98G cells, and Jurkat cells.

In a preferred embodiment, the TRAIL-mediated apoptosis inducing agent is selected from the group comprising encephalitogenic T-cells, soluble TRAIL, TRAIL expressed on the surface of cells, and agonistic antibodies directed against TRAIL-receptor.
In one embodiment, the measurement of an apoptotic response occurs by flow cytometry, immunolabelling with antibodies against active caspase, propidium iodide-fluorescence microscopy and/or electron microscopy, preferably by flow cytometric detection of surface-directed phosphatidyl serine by specific binding of dye-labelled annexin V, flow cytometric detection of DNA-fragmentation by binding of propidium iodide, TUNEL (terminal dUTP nick end labelling), flow cytometric detection of caspase activation by an antibody recognising active caspases and/or immunohistochemical detection of apoptosis by propidium iodide binding.

In a preferred embodiment the method according to the present invention further comprises the step:
f) Selecting those compounds that cause a first apoptotic response which is equal to or less than the second apoptotic response caused by the soluble TRAIL-receptor fusion protein.

The object of the present invention is also solved by a method of screening for compounds capable of inhibiting TRAIL to induce neuronal cell injury comprising the steps:
a) Providing a group of animals A and a group of animals B,
b) inducing the animals to develop experimental autoimmune encephalomyelitis (EAE) by administration of an EAE-inducing agent,
c) administering a compound suspected of being capable of inhibiting TRAIL to induce neuronal cell injury to animals A, and a vehicle substance to animals B,
d) measuring a response to the administration of step c), and
e) comparing the response in group A and group B.
Preferably, the animals of group A and B are mammalian, even more preferably mice.

In one embodiment, the EAE-inducing agent is encephalitogenic T-cells, wherein, more preferably, the encephalitogenic T-cells are PLP-specific.

Preferably, the administration of the EAE-inducing agent in b) occurs by intravenous injection.

In a preferred embodiment, the administration, in c), of a compound suspected of being capable of inhibiting TRAIL occurs by intracistemal and/or intravenous injection.

In one embodiment of the method according to the present invention, in d), a response to the administration step c) is measured by measuring an apoptotic response, wherein, preferably, the measurement of an apoptotic response occurs by flow cytometry, immunolabelling with antibodies against active caspase, propidium iodide-fluorescence microscopy and/or electron microscopy.

The object of the present invention is also solved by a compound identified by the method according to the present invention.

In a preferred embodiment the compound according to the present invention is characterized by its ability to interfere with the interaction of TRAIL and its receptor(s).

Preferably the compound mimicks TRAIL-R2: Fc in its action on TRAIL.

The object is also solved by the use of a compound according to the present invention for the manufacture of a composition for the treatment of multiple sclerosis.

As used herein, the term "TRAIL" refers to tumour necrosis factor-related apoptosis-inducing ligand, either in a free, soluble form or when expressed on the surface of a cell. It encompasses also TRAIL, when expressed as a fusion protein, functional analogues wherein mutations of the aminoacid sequence have been introduced without significantly altering the function or structure of the protein, and truncated forms retaining the biological activity of whole sequence TRAIL. The term "cells which are receptive for TRAIL-mediated apoptosis" is meant to encompass and signify any cell which is susceptible to TRAIL-mediated apoptosis, i.e. which shows apoptotic behaviour upon exposure to TRAIL. The terms TRAIL-R1, TRAIL-R2, ...R3, etc. ...R4 as used herein are to be understood as interchangeable with DR4 (=death receptor4) (for TRAIL-R1), DR5 (for TRAIL-R2), DcR1 (=decoy receptor 1) (for TRAIL-R3), DcR2 (for TRAIL-R4), which are alternative terms sometimes used in the literature. The term "OPG" refers to osteoprotegerin. Usage of any of the aforementioned terms "TRAIL-R1, TRAIL-R2", etc... in connection with ":Fc" is meant to signify a fusion protein of "TRAIL-R1, TRAIL-R2", etc... with the Fc-portion of IgG_{1,} preferably human IgG₁. The term "mimicking" TRAIL-R2:Fc in its action on TRAIL refers to the capability of a compound to act and behave as TRAIL-R2:Fc upon TRAIL. It is currently not known precisely by what actions TRAIL-R2:Fc prevents TRAIL from binding to its cellular receptor. Without wishing to be bound by any theory this may be e.g. by scavenging TRAIL away such that it can not physically interact with its cellular receptor to which it would bind under normal circumstances. As used herein, the term "agonistic antibody directed against TRAIL-receptor" is meant to signify any antibody capable of binding to the TRAIL-receptor(s) and exerting an action thereon that mimics that of TRAIL.

Different models have been proposed as to the functioning of the interaction between TRAIL and its receptors and its physiological role. The present inventors have developed a system, based on their insight into this interaction, which allows to screen for compounds that prove an extremely promising track to follow in the development of medicaments geared towards a treatment of MS.

Reference is now made to the figures, wherein
**Figure 1** shows TRAIL-R2 mRNA expression in selected tissue samples, as assessed by RT-PCR (panel a), TRAIL-R2-protein expression, as assessed by Western blot analysis using two different antibodies (panel b), TRAIL-R2-expression, as analysed by immunohistochemistry in hippocampus (c) or brainstem motor area (d) sections of the murine CNS;
**figure 2** shows a flow cytometric analysis of encephalitogenic PLP-specific CD4+ or CD8+ lymphocytes stained with fluoresceine isothiocyanate-labelled DR5:Fc or isotype control;
**figure 3** shows flow: cytometry results of JURKAT cells that have been incubated with recombinant human TRAIL in the absence or presence of DR5:Fc;
**figure 4** shows the effect of the treatment of DR5:Fc on SJL mice in which chronic adoptive transfer EAE (experimental autoimmune encephalomyelitis) had been induced by intravenous injection of encephalitogenic PLP-specific lymphocytes;
**figure 5** shows transverse brain stem sections immunolabelled with antibody against active caspase 3 from normal, vehicle- or DR5:Fc-treated mice (panel a) sections from a vehicle-treated mouse immunolabeled with antibody against active caspase 3 and counterstained with hematoxylin (panel b), and the average number of neurons positive for active caspase 3 in brain stem section (panel c);
**figure 6** shows propidium iodide-fluorescence microscopy of the CA region in living brain slices without (left upper quadrant) or with the addition of encephalitogenic T-cells (left lower quadrant) (panel a), the CA region in response to application of the neurotoxic agent NMDA as a positive control (right upper quadrant), the inhibition of apoptosis by the TRAIL pathway blocker DR5:Fc (right lower quadrant), the number of PI-positive cells in the neuronal cell layer of CA and DG in the absence or presence of PLP-specific T cells and without or with DR5:Fc and vehicle treatment.

Referring to the figures in detail, figure 1 shows that death-mediating TRAIL-R2 is predominantly expressed on neurons. Brains and spinal cords from naive SJL mice were removed after perfusion with PBS (for RT-PCR and Western blot) or 4% PFA (for immunohistochemistry) and tested for the expression of the mouse TRAIL-R2. ***a***, TRAIL-R2mRNA expression in selected tissue samples including brain and spinal cord were assessed by RT-PCR. ***b***, TRAIL-R2 protein expression assessed by Western blot analysis using two different antibodies. ***c-d,*** TRAIL-R2 expression analyzed by immunohistochemistry in hippocampus (***c***) or brain stem motor area (***d***) sections of the murine CNS. *Arrows* in (***d***) point to cells in the brain stem that are clearly positive. Scale bars 50 µm.

Figure 2 shows that TRAIL is expressed on encephalitogenic lymphocytes. Flow cytometric analysis of encephalitogenic PLP-specific CD4+ or CD8+ lymphocytes stained with fluoresceine isothiocyanate-labeled DR5:Fc or isotype control is demonstrated. Lymphocytes were prepared as used for induction of adoptive transfer EAE or coculture with brain slices (see below) and TRAIL expression was quantified for lymphocyte subpopulations from four independent experiments. *, P<0.05, TRAIL surface staining compared to isotype control staining on CD4+ lymphocytes (Mann-Whitney U-test). Date represent means ± s.e.m.

Figure 3 shows that soluble TRAIL-R2 fusion protein protects from TRAIL-mediated cytotoxicity. To assess functional activity and specificity of soluble TRAIL-R2 fusion protein (DR5:Fc), apoptosis-susceptible Jurkat cells were incubated with recombinant human TRAIL in the absence or presence of DRS:Fc., DNA fragmentation was measured by detection of hypodiploid nuclei via flow cytometry. *, P<0.01 (Mann-Whitney U-test). Data show means ± s.e.m. Other cells that can be used in this type of experiment are cells that are susceptible to TRAIL-mediated apoptosis. These are, for example, LN18 cells and T98G cells which have been shown to be susceptible to TRAIL-mediated apoptosis in Dörr et al., J. Neuroimmunol. 122 (2002), 117-124.

Figure 4 shows a clinical amelioration of chronic autoimmune encephalomyelitis by TRAIL blockade. Chronic adoptive transfer EAE in SJL mice was induced by intravenous injection of encephalitogenic PLP-specific lymphocytes. ***a***, From the time of T cell transfer on, mice were treated with DR5:Fc (*n =* 7; open circles) or control (*n =* 5, ) i.e. twice at the indicated intervals (arrowheads). ***b****,* Mice were treated with DR5:Fc (*n* =5; open circles) or control (*n =* 6, ) in the same way three times at the indicated intervals (arrowheads). ***c***, Mice were treated in the same way with DR5:Fc (*n* = 8; open circles) or control (*n* = 8, ) four times at the indicated intervals (arrowheads). All data represent means ± s.e.m.

Figure 5 shows the inhibition of neuronal damage in autoimmune encephalomyelitis. Active caspase 3 immunoreactive neurons in transverse brain stem sections. ***a***, Sections immunolabeled with antibody against active caspase 3 (amplified with ABC and visualized with HRP/DAB) from normal, vehicle- or DR5:Fc-treated mice. *Arrows* point to those cells that are clearly stained. ***b****,* Section from a vehicle-treated mouse immunolabeled with antibody against active caspase 3 and counterstained with hematoxylin. *Arrows* point to chromatin condensation in a cell positive for active caspase 3. Scale bars 20 µg (***a-b***)*.****c****,* Average number of neurons positive for active caspase 3 in brain stem sections. Neurons were counted; each bar represents three mice from the naive, vehicle-treated, and DR5:Fc-treated groups, with 4 sections analyzed per mouse. *, P<0.01, vehicle-treated compared with DR5:Fc-treated (Mann-Whitney U-test). Data show means ± s.e.m.

Figure 6 shows that inhibition of TRAIL protects from encephalitogenic T cell-mediated neuronal damage. ***a***, Propidium iodide-fluorescence microscopy of the CA region in living brain slices without (left upper quadrant) or with the addition of encephalitogenic T cells (left lower quadrant). As a positive control the CA region is shown in response to application of the neurotoxic agent NMDA (right upper quadrant). Cell death induced by encephalitogenic lymphocytes was inhibited by the TRAIL pathway blocker DR5:Fc (right lower quadrant). Scale bars 100 µm. ***b***, Number of PI-positive cells in the neuronal cell layer of CA and DG in the absence or presence of PLP-specific T cells and without DR5:Fc and vehicle treatment. *n.s.,* non significant; *, P<0.05 (Mann-Whitney U-test). Results are given as means ± s.e.m. indicating cell counts in relation to non-damaged brain tissue (control = 1).

The following examples are intended to illustrate not to limit the scope of the invention. They are merely given by way of example.

### Example 1: Expression of TRAIL-R2 in the murine CNS

### 1.1 Analysis of TRAIL-R2 expression by RT-PCR

Total RNA was isolated from PBS-perfused frozen tissue (brains, spinal cords, liver, thymus and kidney prepared from animals) using the RNAClean™ purification kit (ThermoHybaid, Ulm, Germany). After digestion with DNase I, cDNA was synthesized from 1 µg of total RNA using a commercial RT-PCR beads kit (Pharmacia, Freiburg, Germany). Primer design for mouse TRAIL-R2 was based on the region between cysteine-rich and transmembrane domain of KILLER/DR5 mouse sequence that is not shared by the TNF receptor familiy members known so far (Wu *et al.* 1999, *Cancer Res.* **59**, 2770-2775). PCR conditions were as follows: 30 cycles, 30s/95°C, 30s/65°C, 45s/72°C; TRAIL-R2 primer sequences (Genbank AF176833) 5'-CACAAATACGGTGTGTCGATG-3' (nucleotides 372-392) and 5'-CTGGAACCAGGAGTCCTATCC-3' (nucleotides 551-571); β-actin primer sequences (Genbank J04181) 5'-GTGGGCCGCCCTAGGCACCA-3' (nucleotides 165-184) and 5'-CGGTTGGCCTTAGGGTTCAGGGGG-3' (nucleotides 386-409). Isolated PCR fragments were reamplified and checked with ABI 377 DNA sequencing system (Applied Biosystems, Weiterstadt, Germany).

As shown in *Fig. 1a,* analysis by RT-PCR technique revealed a clear signal for TRAIL-R2 in the brain and spinal cord of SJL mice as well as in control tissue.

### 1.2: Analysis of TRAIL-R2 protein expression by Western blot

PBS-perfused frozen tissue was homogenized in extraction buffer containing 1% Triton X-100 and protease inhibitors (0.2 mM PMSF, 1 µg/ml pepstatin, 0.5 µg/ml leupeptin). Samples were separated by 12% SDS-PAGE and blotted onto a nitrocellulose membrane. Following overnight blocking at 4°C in a blocking cocktail with 5% fat-free instant milk, membranes were incubated with anti-TRAIL-R2 antibodies for 2 h at room temperature (dilution 1:1000, Ab1687 or Ab16942; Chemicon, Temecula, California). Membranes were washed, incubated for 1 h with secondary antibodies coupled to horse radish peroxidase (Dako Diagnostica, Hamburg, Germany), washed again, and developed with the ECL-plus system (Pharmacia). Membranes were sequentially incubated with an antibody against β-actin (1:20,000; Sigma, Deisenhofen, Germany), washed, incubated for 1 h with rabbit anti-mouse antibody (1:5,000; Dako), and developed.

Antibody Ab 16942 was previously tested for specificity on transfected CV-1 EBNA cells selectively expressing TRAIL-R2 (Dörr *et al.* 2002, *J Neurosci.* **22**, RC209).

As demonstrated in *Fig. 1b,* Western blot analysis of brain lysates confirmed the initial PCR results (*cf. Example 1.1.*).

### 1.3: Analysis of TRAIL-R2 protein expression by immunohistochemistry

Mice euthanized with narcotics were transcardially perfused with 4% paraformaldehye (PFA). Horizontal 50-mm-thick vibratome sections (Shandon, Pittsburgh, Pennsylvania) were immersed with 3% H₂O₂ to quench unspecific endogenous peroxidas activity, washed, and blocked (10% normal goat serum and 0.5% Triton X-100) prior to overnight incubation with anti-TRAIL-R2-antibody (Ab16942, 1:50, Chemicon). Sections were immunolabeled using the avidin:biotinylated enzyme complex technique (ABC-Elite, Vector Laboratories, Burlingame, California) before development with diaminobenzidine as substrate.

Immunohistochemical staining with Ab16942 (*Fig. 1c-d*) showed a characteristic distribution with predominantly neurons expressing the death-mediating TRAIL-R2, as demonstrated for hippocampal and brain stem sections.

### Example 2: Expression of TRAIL on activated lymphocytes

Analysis of TRAIL expression in encephalitogenic lymphocytes (primary lymph node cell cultures specifically stimulated with myelin components and prepared following established protocols, see Steinbrecher et al., *J. Immunol.* **166**, 2041-2048), as they are used for adoptive transfer EAE, was analyzed using a fluorescein isothiocyanate (FITC)-labeled soluble TRAIL-R2 in flow cytometry. T cells were harvested and activated for 4 h with 5 ng/ml PMA and 1µg/ml ionomyxin (Sigma) as described (Mariani & Krammer 1998, *Eur. J. Immunol.* **28**, 1492-1498). Cells were initially blocked with rat anti-mouse CD16/CD32 antibody (to exclude FcγII/III-mediated unspecific interactions) prior to staining for CD4 (with a combination of a biotin-conjugated anti-mouse CD4 and a streptavidine-CyChrome® -conjugate; both BD Pharmingen, Heidelberg, Germany), for CD8 (phycoerithrin-conjugated antibody against mouse CD8; BD Pharmingen), and for TRAIL (FITC-labeled DR5:Fc; Alexis, San Diego, California). We analyzed 50,000 cells by three color flow cytometry on a FACS-Calibur® (BD Pharmingen).

As demonstrated in *Fig. 2,* TRAIL expression of PLP-specific lymphocytes was primarily resc-tricted to the CD4+ subpopublation, which make up 60% of the transferred encephalitogenic lymphocytes, with the CD8+ proportion revealed almost no TRAIL expression.

### Example 3: Inhibition of EAE by TRAIL blockade

Blocking experiments were performed in the adoptive transfer EAE model induced by PLP-specific T cells in SJL mice. All procedures were conducted according to protocols approved by the local animal welfare committees.

First, the activity of soluble TRAIL-R2 fusion protein (DR5:Fc ( = TRAIL-R2:Fc); Alexis, San Diego, USA) was tested. Jurkat cells (2x10⁵) were incubated with rhTRAIL (with 2µg/ml enhancer; Alexis) in the presence or absence of 0.5 µg/ml DR5:Fc (Alexis). After 24 h, cells were washed and incubated overnight at 4°C in a hypotonic fluorochrome solution containing 50 µg/ml propidium iodide prior to flow cytometry for quantitative detection of hypodiploid DNA (Nicoletti *et al.* 1991, *J. Immunol. Methods* **139**, 271-279). Other fusion proteins of TRAIL-receptors, for example with the Fc-portion of human IgG1 may be used, e.g. TRAIL-R1:Fc, TRAIL-R2:Fc, TRAIL-R3:Fc, TRAIL―R4:Fc or OPG:Fc (OPG = Osteoprotegerin).

As shown in *Fig. 3,* DR5:Fc was found to be functionally active and completely protected Jurkat cells from TRAIL-mediated cytotoxicity.

Next, female SJL recipient mice (6-8 weeks; Harlan, Rehovot, Israel; Charles-River, Sulzfeld, Germany) were injected i.v. with 3x10⁷ activated PLP139-151-specific lymph node cells (LNC) derived from syngeneic donors and cultured with antigen for 4 days prior to transfer. The immunization of donor mice with murine PLP139-151 (purity >95%; Peceuticals, Leicester, UK) and preparation of primary LNC cultures followed established protocols (Steinbrecher *et al.* 2001, *J. Immunol.* **166**, 2941-2048). Antigen-specificity of injected cells was routinely checked with a standard [³H]thymidine proliferation assay. Neurological deficits were graded as described (Steinbrecher *et al.* 2001, *J. Immunol.* **166**, 2941-2048). Within 7 to 10 days after cell transfer, these mice start to develop disease with marked motor weakness and incontinence. Although the acute attack is followed by clinical remission, animals normally show an incomplete recovery with long-term neurological deficits.

For treatment with DR5:Fc, mice were anesthetized with ketamine (50 mg/kg; Curamed, Karlsruhe, Germany) and xylazine (10 mg/kg; Bayer, Leverkusen, Germany) before puncture of the cistema magna with a 26 gauge syringe (Hamilton, Bonduz, Switzerland) and injection of DR5:Fc (1 µg per mouse) or control (1 µg human Fc fragment dialyzed against PBS; Calbiochem, San Diego, California; or PBS for EAE in *Fig. 4b*) in a volume of 10 µl (Meyding-Lamadé *et al.* 1996, *J. Exp. Anim. Sci.* **38**, 77-81). Intracisternally (i.c.) injection was at several time points from the lymphocyte transfer on.

Although the onset of passive EAE was slightly accelerated, the DR5:Fc-treated mice, as compared to the control-injected animals, had markedly decreased clinical signs thereafter as demonstrated in *Fig. 4a-c.* The protective effect of TRAIL blockade was dose-dependent, since an increased number of injections showed accordingly more clinical efficacy.

### Example 4: Inhibition of neuronal injury by TRAIL blockade in vivo

Animals from the EAE experiment (*cf. Example 3*) were sacrificed on day 20 and immunohistochemistry was performed as outlined above (*cf. Example 1.3.*)*.* Antibody incubation was by using anti-caspase-3 antibody (1:100; R&D, Wiesbaden, Germany). For counterstaining of nuclei, brain stem sections stained for active caspase-3 were incubated with Harris' hematoxylin for 1 min at room temperature.

Staining for active caspase-3 revealed positive neurons with transected neurites predominantly localized in central motor areas (*Fig. 5a*). Activation of caspase was regularly accompanied by chromatin condensation, as detected by hematoxylin counterstaining (*Fig. 5b*). Since apoptotic neurons in normal control mice are entirely absent, the appearance of apoptotic neurons present a pivotal step towards the development of long-term neurological disability. In line with the clinical data, quantitative assessment of caspase-positive neurons indicated a significant difference between DRS:Fc- or vehicle-treated animals (*Fig. 5c*), despite the comparable amount of inflammation in these groups.

### Example 5: Inhibition of neuronal injury by TRAIL blockade in vitro

To confirm the contribution of the TRAIL pathway to neuronal damage and to gain mechanistic insight, the direct effects of encephalitogenic lymphocytes on living organotypic brain slices from syngeneic mice were studied. For that purpose, organotypic entorhinal-hippocampal slice (350 µm thick) cultures from 10-day-old SJL mice were prepared on a tissue chopper (Bachhofer, Reutlingen, Germany) and cultured as described (Ulrich *et al.* 2001, *Nat. Cell. Biol.* **3**, 1035-1042). For co-culture, 5x10⁴ encephalitogenic lymphocytes (as used for EAE induction; *cf. Example 3*) were prelabled with carboxyfluorescein diacetate (CFDA) (Gimsa *et al.* 2000, *Brain Pathol.* **10**, 365-377) and transferred to the surface of slices (day 7 *in vitro*) preincubated with DR5:Fc or Fc fragment alone (both 1 µg/ml) for at least 3 h. As positive control, 50µM N-methyl-D-aspartate (NMDA) were applied. Damage in neuronal cell layers of the comu ammonis (CA) and granule cell layer of the dentate gyrus (DG) was detected 12-24 h thereafter by addition of 5 µg/ml propidium iodide (30 min at 36°C) (Ulrich *et al.* 2001, *Nat. Cell. Biol.* **3***,* 1035-1042). Slices were wahsed and examined using a rhodamine filter (488 nm/515 nm) with a dark-field inverse fluorescence microscope (Olympus, Tokyo, Japan). Dead lymphocytes were distinguished by double-fluorescence microscopy and subtracted from the counts of propidium iodide-positive cells within the neuronal layers of the slices. For resectioning, slices were immersed prior to the preparation of 14 µm thin horizontal cryostat sections, and quantification was carried out as described elsewhere (Ulrich *et al.* 2001, *Nat. Cell. Biol.* **3**, 1035-1042). Encephalitogenicity of PLP-specific lymphocytes was routinely checked by successful induction of adoptive transfer EAE.

In *Fig. 6 a, b* it is shown that cell death induced by encephalitogenic lymphocytes was inhibited by DR5:Fc. Moreover, the number of propidium iodide-positive cells in the neuronal layer of CA and DG induced by PLP-restiumulated lymphocytes was significantly reduced to a background level via blockade with the soluble DR5:Fc (*cf. Fig 6b*). Induction of cell death (as visualized by propidium iodide staining in *Fig. 6a*) by invading T cells was only found when these cells expressed TRAIL.

The features disclosed in the foregoing description, in the claims and/or in the accompanying drawings may, both separately and in any combination thereof, be material for realising the invention in diverse forms thereof.

## Claims

1. A method of screening for compounds capable of inhibiting tumor necrosis factor-related apoptosis inducing ligand (TRAIL) to induce neuronal cell injury comprising the steps:
a) contacting TRAIL with a compound/compounds/a combinatorial library of compounds suspected of inhibiting TRAIL,
b) detecting an interaction of TRAIL with a compound/compounds.

2. A method according to claim 1 further comprising the step
c) selecting those compounds that detectably interact with TRAIL.

3. A method according to any of claims 1-2, **characterised in that** detection occurs by a method selected from the group comprising fluorimetry, surface plasmon resonance, gel filtration chromatography, mass spectrometry and nuclear magnetic resonance (NMR).

4. A method of screening for compounds capable of interfering with the interaction of TRAIL and its receptor(s) comprising the steps:
a) Contacting cells which are receptive for TRAIL-mediated apoptosis with a compound suspected of interfering with the interaction of TRAIL with its receptor(s) in the presence of a TRAIL-mediated apoptosis inducing agent,
b) measuring an apoptotic response.

5. A method according to claim 4 further comprising the steps:
c) Contacting cells of the same type as in a) but not having undergone the contacting step a), with a soluble TRAIL-receptor-fusion protein in the presence of a TRAIL-mediated apoptosis inducing agent,
d) measuring an apoptotic response,
e) comparing the apoptotic response of step b) with the apoptotic response of step d).

6. A method according to any of claims 4 and 5, **characterised in that** the cells contacted in steps a) and c) are cell slices of tissue, cell suspensions and/or cells grown on a surface.

7. A method according to claim 6 **characterised in that** the surface is an artificial surface.

8. A method according to claim 7 **characterised in that** the artificial surface is a surface of a membrane of a bioreactor.

9. A method according to any of claims 5-8, **characterised in that** the soluble TRAIL-receptor fusion protein is selected from the group comprising TRAIL-R1 :Fc, TRAIL-R2:Fc, TRAIL-R3:Fc, TRAIL-R4:Fc and OPG:Fc.

10. A method according to any of claims 4-9 **characterised in that** the cells which are receptive for TRAIL-mediated apoptosis are selected from the group comprising neuronal cell lines, glioma cell lines, in particular LN18 cells and T98G cells, and Jurkat cells.

11. A method according to any of claims 4-10, **characterised in that** the TRAIL-mediated apoptosis inducing agent is selected from the group comprising encephalitogenic T-cells, soluble TRAIL, TRAIL expressed on the surface of cells, and agonistic antibodies directed against TRAIL-receptor.

12. A method according to any of claims 4-11, **characterised in that** the measurement of an apoptotic response occurs by flow cytometry, immunolabelling with antibodies against active caspase, propidium iodide-fluorescence microscopy and/or electron microscopy.

13. A method according to claim 12, **characterised in that** the measurement of an apoptotic response occurs by flow cytometric detection of surface-directed phosphatidyl serine by specific binding of dye-labelled annexin V, flow cytometric detection of DNA-fragmentation by binding of propidium iodide, TUNEL (terminal dUTP nick and labelling), flow cytometric detection of caspase activation by an antibody recognising active caspases and/or immunohistochemical detection of apoptosis by propidium iodide binding.

14. A method according to any of claims 5-13 further comprising the step:
f) Selecting those compounds that cause a first apoptotic response which is equal to or less than the second apoptotic response caused by the soluble TRAIL-receptor fusion protein.

15. A method of screening for compounds capable of inhibiting TRAIL to induce neuronal cell injury comprising the steps:
a) Providing a group of animals A and a group of animals B,
b) inducing the animals to develop experimental autoimmune encephalomyelitis (EAE) by administration of an EAE-inducing agent,
c) administering a compound suspected of being capable of inhibiting TRAIL to induce neuronal cell-injury to animals A, and a vehicle substance to animals B,
d) measuring a response to the administration of step c), and
e) comparing the response in group A and group B.

16. A method according to claim 15, **characterised in that** the animals of group A and B are mammalian.

17. A method according to claim 16, **characterised in that** the animals are mice.

18. A method according to any of claims 15-17, **characterised in that** the EAE-inducing agent is encephalitogenic T-cells.

19. A method according to claim 18, **characterised in that** the encephalitogenic T-cells are PLP-specific.

20. A method according to any of claims 15-19, **characterised in that** the administration of the EAE-inducing agent in b) occurs by intravenous injection.

21. A method according to any of claims 15-20, **characterised in that** the administration of a compound suspected of being capable of inhibiting TRAIL in c) occurs by intracis-ternal and/or intravenous injection.

22. A method according to any of claim 15-21, **characterised in that**, in d), a response to the administration step c) is measured by measuring an apoptotic response.

23. A method according to claim 22, **characterised in that** the measurement of an apoptotic response occurs by flow cytometry, immunolabelling with antibodies against active caspase, propidium iodide-fluorescence microscopy and/or electron microscopy.

24. A compound identified by the method according to any of the foregoing claims.

25. A compound according to claim 24, **characterised by** its ability to interfere with the interaction of TRAIL and its receptor(s).

26. A compound according to any of claims 24-25, mimicking TRAIL-R2:Fc in its action on TRAIL.

27. Use of a compound according to any of claim 24-26 for the manufacture of a composition for the treatment of multiple sclerosis.
